# EUROPEAN PATENT SPECIFICATION

(11) **EP 4 249 376 B1**
(45) Date of publication and mention of the grant of the patent: **03.09.2025**
(21) Application number: 23162307.5
(22) Date of filing: 16.03.2023
(51) Int. Cl.: B64D 11/04, A47J 31/60, A47J 31/44, B67D 1/07, B67D 1/08, B67D 1/12

(54) **SANITIZATION SYSTEMS AND METHODS FOR BEVERAGE DEVICES**
DESINFEKTIONSSYSTEME UND -VERFAHREN FÜR GETRÄNKEVORRICHTUNGEN
SYSTÈMES ET PROCÉDÉS DE DÉSINFECTION POUR DISPOSITIFS DE BOISSON

(30) Priority: 22.03.2022 US 202217700830
(43) Date of publication of application: 27.09.2023
(73) Proprietor: B/E Aerospace, Inc., Winston-Salem, NC 27105 (US)
(72) Inventor: SPINNER, JACOB A., Overland Park (US); ROGERS, BRYAN N., Kearney (US)
(74) Representative: Dehns

(56) References cited:
- WO-A1-2019/112123
- WO-A1-2022/015451
- CN-A- 101 431 928
- CN-A- 108 185 823

## Description

### FIELD

The present disclosure relates generally to beverage devices, systems, and methods, and, more particularly, to sanitization systems and methods for use in beverage devices.

### BACKGROUND

Pathogens can be transmitted via direct airborne transmission between users or via indirect contact transmission from different users occupying the same space at different times. For example, lingering pathogens may remain on contact surfaces of an aircraft cabin to be spread to passengers and/or crew members on a subsequent flight. The safety of passengers and crew members may be improved by performing disinfecting treatments to surfaces, such as seats, ceiling/wall panels, handles, and lavatory surfaces, beverage devices, etc., to mitigate the presence of pathogens on such surfaces. However, conventional disinfection procedures between flights may take time and may thus adversely affect the operating efficiency of the aircraft (increased interval time between flights), and the effectiveness and quality of such conventional treatments are often difficult to verify/track.

For aerospace beverage makers, the water is heated more than 170 degrees Fahrenheit (or 77 degrees Celsius) for over 30 seconds thus providing thermal disinfection for all surfaces in contact. However, current aerospace beverage service equipment does not have any integrated devices for the explicit purpose of disinfecting the product or beverage from bacteria and viruses. The devices are used in every flight by the flight attendants and each touch interaction potentially introduces bacteria and viruses. In addition, airborne contaminants can also be introduced onto the devices, potentially passing the contaminant to the passengers.
CN101431928A relates generally to beverage makers and, more particularly, to beverage makers constructed on transport vehicles including commercial aircraft. The beverage maker has an in-line heating assembly for heating water to make hot beverages such as coffee and tea. An optical sensor assembly is provided to detect when a serving container of the beverage maker has a prescribed capacity of liquid within it. The sensor assembly includes at least one light source aligned to shine into the container and at least one detector aligned to detect light reflected off the liquid surface within the container.

CN108185823A relates to an anti-overflow water dispenser.

WO2019/112123A1 relates to a water purifier. The water purifier includes a water purifier body including a housing defining an outer appearance, and a filter provided inside the housing to filter raw water introduced from the outside. A water discharging nozzle is exposed to a front surface of the water purifier body and is configured to supply water passing through the filter to the outside of the water purifier body. An ultraviolet lamp is provided above the water discharging nozzle and is configured to emit ultraviolet rays to an inner surface of the water discharging nozzle.

WO 2022/015451 A1 relates to a contactless autofill dispenser. The contactless autofill dispenser includes a housing and a dispense area for receiving a cup. The dispenser includes a supply of consumable product disposed within the housing. The dispenser includes an outlet connected to the supply and extending from the housing into the dispense area for dispensing the consumable product into the cup. The dispenser includes a controller disposed within the housing and connected to the supply, wherein the controller is configured to control dispensing of the consumable product from the outlet. The dispenser includes a time of flight sensor attached to or with a direct line of sight into the dispense area and connected to the controller.

### SUMMARY

A beverage maker is provided as claimed in claim 1. The beverage maker can comprise: a housing defining a recess, the recess configured to receive a server in the recess; and a sanitization device disposed in the recess, the sanitization device comprising an ultraviolet light emitter and an ultraviolet light sensor, the ultraviolet light emitter oriented towards a base of the housing, the sanitization device configured to detect a fluid level in the server during dispensing of a fluid and configured to at least partially sanitize one of the server and the fluid during dispensing of the fluid and/or the recess in response to the server not being present.

In various embodiments, the beverage maker further comprises a controller in electronic communication with the ultraviolet light emitter and the ultraviolet light sensor, the controller configured to: receive an input signal; couple a power supply to the ultraviolet light emitter in response to receiving the input signal; receive sensor data from the ultraviolet light sensor; and decouple the power supply from the ultraviolet light emitter in response to determining an ultraviolet radiation level exceeded an ultraviolet radiation threshold.

In various embodiments, the beverage maker further comprises a second ultraviolet radiation emitter disposed in the housing and oriented towards surfaces within the recess. In various embodiments, the coffee or beverage maker further comprises a controller in electronic communication with the second ultraviolet radiation emitter, the controller configured to: receive an input signal; couple a power supply to the second ultraviolet radiation emitter in response to receiving the input signal; and decouple the power supply from the second ultraviolet radiation emitter in response to exceeding a time threshold.

In various embodiments, the ultraviolet light emitter is configured to emit ultraviolet radiation having an average wavelength between 100 nm and 400 nm. The average wavelength can be between 200 nm and 400 nm or between 200 nm and 280 nm.

A retrofit process for a beverage dispensing device is provided as claimed in claim 6. The retrofit process can comprise: decoupling a level sensor from a location of a housing of the beverage dispensing device, the level sensor configured to detect a level of a fluid in a container during a dispensing process of the beverage dispensing device; and coupling a sanitization device in the location of the housing, the sanitization device comprising an ultraviolet light emitter and an ultraviolet light sensor, the sanitization device configured to detect the level of the fluid in the container during the dispensing process of the beverage dispensing device and sanitize one of the beverage dispensing device and the container.

In various embodiments, the ultraviolet light emitter is configured to emit ultraviolet radiation having an average wavelength between 100 nm and 400 nm or between 200 nm and 400 nm.

In various embodiments, the level sensor is configured to emit infrared radiation having an average wavelength between 780 nm and 1 mm.

In various embodiments, the level sensor comprises a first housing having a first shape and the sanitization device comprises a second housing having a second shape, and wherein, the second shape is substantially similar to the first shape.

The forgoing features and elements may be combined in various combinations without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the following detailed description and claims in connection with the following drawings. While the drawings illustrate various embodiments employing the principles described herein, the drawings do not limit the scope of the claims.
FIG. 1 illustrates a view of a cabin of an aircraft, in accordance with various embodiments;
FIG. 2A illustrates a beverage dispensing device having a sanitization system, in accordance with various embodiments;
FIG. 2B illustrates a schematic view of the beverage dispensing device of FIG. 2A, in accordance with various embodiments;
FIG. 2C illustrates the beverage dispensing device of FIG. 2A in use, in accordance with various embodiments;
FIG. 3 illustrates a beverage dispensing device having a sanitization system, in accordance with various embodiments;
FIG. 4A illustrates a beginning of a retrofit process, in accordance with various embodiments;
FIG. 4B illustrates a middle of a retrofit process, in accordance with various embodiments;
FIG. 4C illustrates an end of a retrofit process, in accordance with various embodiments;
FIG. 5 illustrates a flow chart for a retrofit process, in accordance with various embodiments.

### DETAILED DESCRIPTION

The following detailed description of various embodiments herein refers to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the claims. Thus, the detailed description herein is presented for purposes of illustration only and not of limitation. Also, any reference to attached, fixed, connected, or the like may include permanent, removable, temporary, partial, full or any other possible attachment option.

Disclosed herein is sanitization system including an integrated light emitting source (e.g., ultraviolet light) having a wavelength range configured to safely disinfect a beverage device and/or a beverage container in a location where the beverage(s) are dispensed. As described further herein, the sanitization system can be located in a cavity or faucet area where a service cup or server is located (i.e., any beverage service devices, such as soda dispensers, water dispensers, coffee and/or tea makers, etc.). For a beverage maker, a sanitization system may be retrofitted into an existing beverage maker by replacing a liquid level detector (e.g., an infrared LEDs/photo-receivers) with a sanitization device (e.g., a combined sanitization and level detection system). The liquid level detector may comprise a disc that is disposed vertically above the coffee server. A retrofit process comprises removing the liquid level detector from the beverage maker and installing the sanitization device. In various embodiments, the sanitization device comprises an ultraviolet light emitter (e.g., emitting radiation with an average wavelength between 100 nanometers (nm) and 400 nm, or between 200 nm and 330 nm, or between 200 nm and 280 nm) and an ultraviolet light sensor.

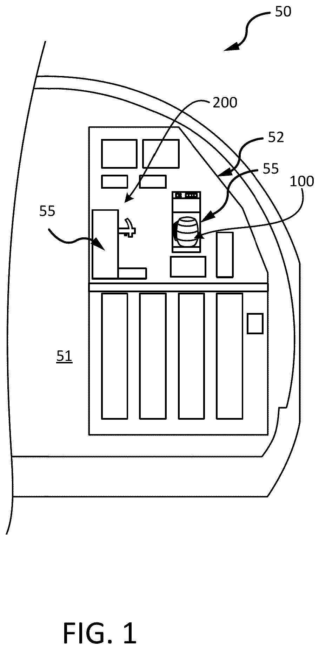

With reference to FIG. 1, a cabin 51 of an aircraft 50 is shown, according to various embodiments. The aircraft 50 may be any aircraft such as an airplane, a helicopter, or any other aircraft. The aircraft 50 defines an aircraft cabin 51 in a fuselage of the aircraft 50. Disposed in the aircraft cabin 51 is a galley 52 (or server station). The galley 52 includes various beverage devices 55 (e.g., a coffee maker 100, a beverage dispensing device 200, or the like). Although described herein with respect to the coffee maker 100 and the beverage dispensing device 200, the present disclosure is not limited in this regard. For example, any beverage device 55 with a sanitization system for use in an aircraft gallery 52 or the like is within the scope of this disclosure. Although described herein with respect to the galley 52 in an aircraft cabin, the present disclosure is not limited in this regard. For example, the beverage devices 55 may be disposed on a beverage cart in an aircraft cabin, or any other location where sanitization around a beverage device is desired.

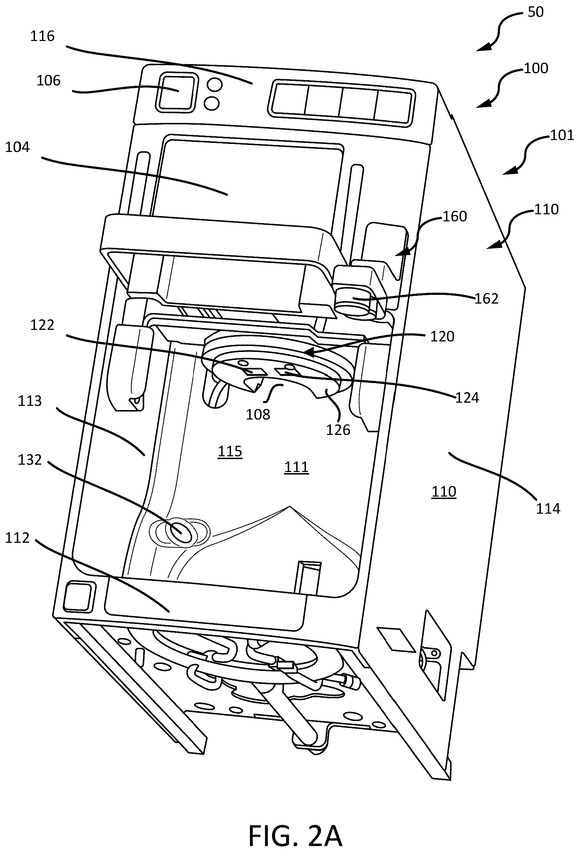

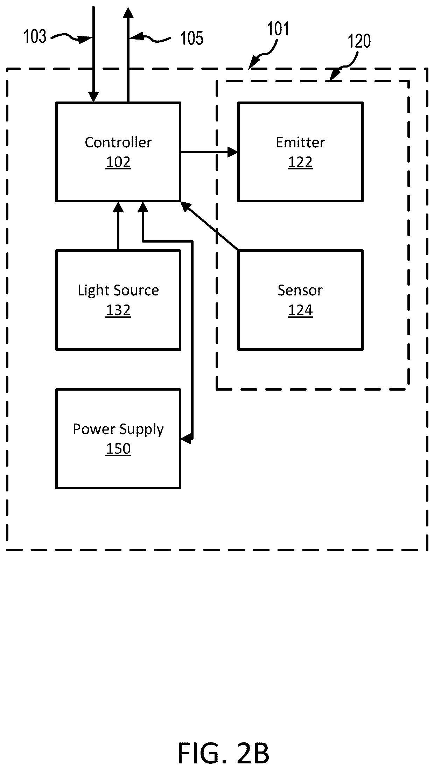

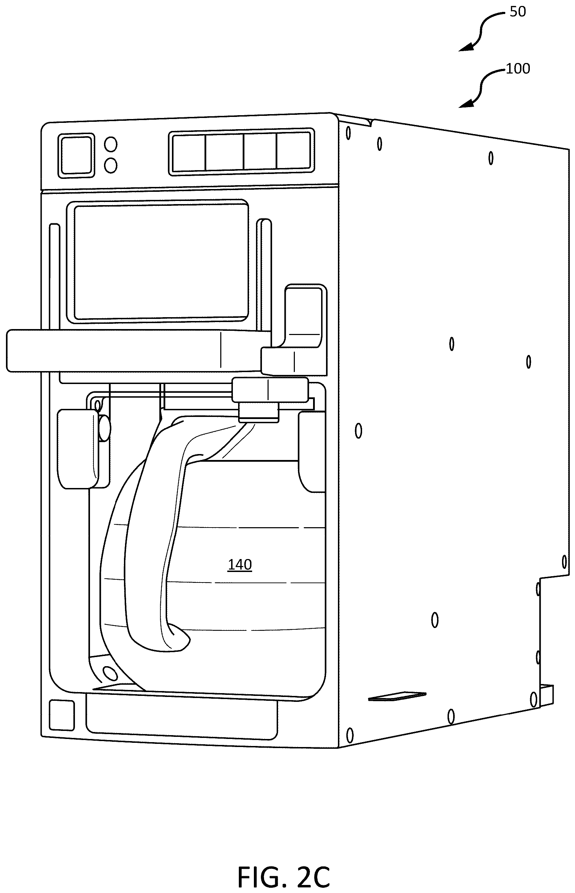

Referring now to FIGs. 2A and 2B, a beverage device 55 (e.g., coffee or tea maker 100, referred to herein as a "beverage maker") having a sanitization system 101 for use in the galley 52 or a beverage cart on an aircraft 50 from FIG. 1, is illustrated, in accordance with various embodiments. The beverage maker 100 comprises a housing 110, and the sanitization system 101. The sanitization system 101 includes a sanitization device 120. The housing 110 comprises a base 112, sidewalls 113, 114, 115 and a top portion 116. The top portion 116 may include a lid, configured to open, and receive a filter and filter basket therein. The housing 110 can further comprise a reservoir configured to receive water therein. In various embodiments, the base 112, sidewalls 113, 114, 115 and the top portion 116 define a recess 111. The recess 111 is configured to receive a server (e.g., a coffee server 140 from FIG. 2C), as described further herein. In various embodiments, beverage maker 100 further comprises a feed spout 108 in fluid communication with a reservoir disposed within the housing 110 during operation. In this regard, coffee can be dispensed from the beverage maker 100 via the feed spout 108 during operation, in accordance with various embodiments.

In various embodiments, the sanitization device 120 is disposed within the recess 111 proximal (i.e., adjacent to, or abutting), the top portion 116 of the beverage maker 100. The sanitization device 120 comprises an ultraviolet light emitter 122, and an ultraviolet light sensor 124. In various embodiments, the ultraviolet light emitter 122 is configured to emit radiation with an average wavelength between 100 nm and 400 nm, or between 200 nm and 330 nm, or between 200 nm and 280 nm (referred to herein as "ultraviolet radiation"). In various embodiments, the ultraviolet light emitter 122 is configured to emit radiation with an average wavelength between 100 nm and 280 nm (e.g., UV-C light). In this regard, the ultraviolet light emitter 122 can be configured to emit light for sanitizing surrounding components and surfaces that are contacted by the emitted radiation. In various embodiments, the ultraviolet light sensor 124 is configured to measure a photon flux density and/or an energy flux density of ultraviolet radiation.

Although illustrated as including a single ultraviolet light emitter 122, the present disclosure is not limited in this regard. For example, the sanitization device 120 may comprise any number of the ultraviolet light emitter 122 and be within the scope of this disclosure.

In various embodiments, the sanitization device 120 is configured to sanitize a server (e.g., server 140 from FIG. 2C), sanitize a liquid in the server 140, and/or detect a fluid level within the server 140. In this regard, the sanitization device 120 can replace a typical level sensor of a beverage maker 100 and provide an additional benefit of sanitization, in accordance with various embodiments.

In various embodiments, in response to having a plurality of light emitters (e.g., the ultraviolet light emitter 122), only a single light emitter in the plurality of light emitters may be oriented to reflect light back to the ultraviolet light sensor 124. In this regard, a single pair of an ultraviolet light emitter 122 and an ultraviolet light sensor 124 can work together to provide fluid level detection and sanitization, and the additional ultraviolet light emitters 122 may provide additional sanitization (e.g., of the recess 111 of the housing 110, outer surfaces of the server 140, or the like). The present disclosure is not limited in this regard.

In various embodiments, the ultraviolet light emitter 122 and ultraviolet light sensor 124 are disposed within a housing 126 of the sanitization device 120. The housing 126 may be configured to be installed in a beverage maker 100 as illustrated in FIG. 2A.

In various embodiments, the ultraviolet light emitter 122 and the ultraviolet light sensor 124 are in electronic (e.g., electrical, or wireless) communication with a controller 102. In various embodiments, the controller 102 can be integrated into a microcontroller. In various embodiments, controller 102 can be configured as a central network element or hub to access various systems and components of the sanitization system 101 and/or the beverage maker 100. For example, controller 102 can be a main controller of the beverage maker 100 or controller 102 can be a local controller configured to interface with a main controller of a beverage maker 100. The present disclosure is not limited in this regard.

Controller 102 can comprise a network, computer-based system, and/or software components configured to provide an access point to various systems and components of sanitization system 101. In various embodiments, controller 102 comprises a processor. In various embodiments, controller 102 is implemented in a single processor. In various embodiments, controller 102 is implemented as and may include one or more processors and/or one or more tangible, non-transitory memories and be capable of implementing logic. Each processor can be a general purpose processor, a digital signal processor (DSP), an application specific integrated circuit (ASIC), a field programmable gate array (FPGA) or other programmable logic device, discrete gate or transistor logic, discrete hardware components, or any combination thereof. Controller 102 can comprise a processor configured to implement various logical operations in response to execution of instructions, for example, instructions stored on a non-transitory, tangible, computer-readable medium configured to communicate with controller 102.

System program instructions and/or controller instructions may be loaded onto a non-transitory, tangible computer-readable medium having instructions stored thereon that, in response to execution by a controller, cause the controller to perform various operations. The term "non-transitory" is to be understood to remove only propagating transitory signals per se from the claim scope and does not relinquish rights to all standard computer-readable media that are not only propagating transitory signals per se. Stated another way, the meaning of the term "non-transitory computer-readable medium" and "non-transitory computer-readable storage medium" should be construed to exclude only those types of transitory computer-readable media which were found in *In Re Nuijten* to fall outside the scope of patentable subject matter under 35 U.S.C. § 101.

In various embodiments, the controller 102 is configured to receive an input signal 103. For example, the input signal 103 can be from a sensor (e.g., a motion sensor, a weight sensor, or the like) indicating that a server 140 from FIG. 2C has been placed in the recess 111. In various embodiments, the input signal 103 may be from a button 104 disposed on the housing 110 being pressed (e.g., to start a brew process for coffee in the beverage maker 100, or the like). The present disclosure is not limited in this regard.

In response to receiving the input signal 103, the controller 102 can electrically couple a power supply (e.g., via a switch, or the like), to the ultraviolet light emitter 122. In this regard, ultraviolet radiation can be emitted from the ultraviolet light emitter 122 for an entire duration of a brewing process, for a brewing process and after the brewing process for a predetermined period of time (e.g., to clean the recess 111 of the housing 110).

In various embodiments, while sanitizing in accordance with the above, the ultraviolet light sensor 124 is receiving measurements of photon flux density and/or an energy flux density of ultraviolet radiation and relaying the sensor data to the controller 102. In various embodiments, based on the sensor data, the controller 102 may be configured to determine a level of fluid in the coffee server 140 from FIG. 2C, determine an amount of ultraviolet radiation emitted in the recess 111.

In various embodiments, in response to the controller determining a threshold fluid level in the server 140 has been met or exceeded, the controller 102 can send an output signal 105 to terminate a coffee brewing process. In this regard, based on the sensor data from the ultraviolet light sensor 124, the controller 102 can determine a fluid level in the server 140 from FIG. 2C, compare the fluid level to a threshold level, and command termination of a brewing process by the beverage maker 100 in response to the fluid level exceeding the threshold level.

In various embodiments, the controller 102 may further be configured to monitor an amount of ultraviolet radiation released at one time. For example, excessive exposure to ultraviolet radiation may have negative health effects when exposed to human skin. In this regard, the controller 102 can be further configured to decouple the power supply 150 from the ultraviolet light emitter 122 in response to determining an amount of ultraviolet radiation has exceeded a threshold amount of radiation. In various embodiments, an amount of radiation may be measured in photon flux density and/or an energy flux density of ultraviolet radiation. The present disclosure is not limited in this regard.

In various embodiments, the sanitization system 101 can further comprise an ultraviolet light emitter 122 separate from the ultraviolet light emitter 122 of the sanitization device 120. In this regard, the ultraviolet light emitter 132 can be configured to sanitize the sidewalls 113, 114, 115 and the base 112 within the recess 111 periodically, after a brewing process, or the like. In various embodiments, the ultraviolet light emitter 132 can also be configured to sanitize an outer surface of the server 140 from FIG. 2C. In this regard, the controller 102 can be in electronic communication with the ultraviolet light emitter 132 and configured to couple the ultraviolet light emitter 132 to the power supply 150 in a similar manner as outlined above.

In various embodiments, as described further herein, the sanitization system 101 can comprise the sanitization device 120 and/or the ultraviolet light sensor 132. In this regard, a sanitization system 101 including only the sanitization device 120, including only the ultraviolet light sensor 132, or including both the sanitization device 120 and the ultraviolet light sensor 132 are all within the scope of this disclosure.

For example, in response to the controller 102 receiving an input signal 103 (e.g., from a button 106, from a main controller of the beverage maker 100, from a motion sensor or a weight sensor, etc.), the controller 102 can couple the power supply 150 to the ultraviolet light emitter 132. In various embodiments, the controller 102 can be configured to emit a predetermined amount of ultraviolet light from the light source 132 (e.g., for a predetermined amount of time or the like). In this regard, the controller 102 can control how much ultraviolet radiation is emitted from the ultraviolet light emitter 132 during sanitization of the recess 111.

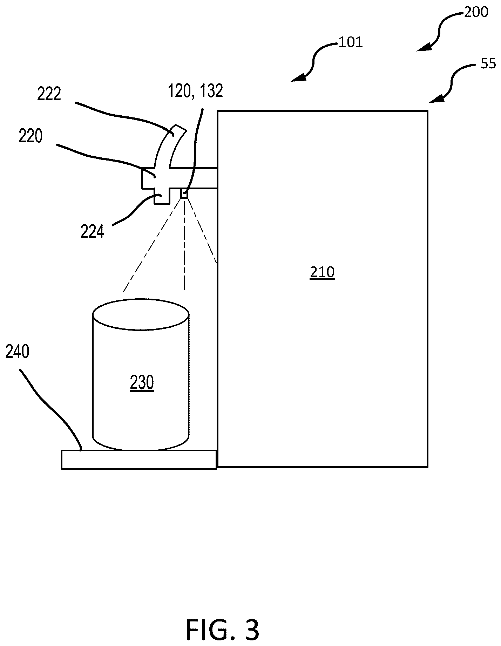

Although described herein with respect to a beverage maker 100, the sanitization system 101 is not limited in this regard. For example, a sanitization system 101 can be used in other beverage devices for use in an aircraft 50 from FIG. 1, such as a beverage dispensing device 200 as shown in FIG. 3. With reference now to FIG. 3, the beverage dispensing device 200 comprises a housing 210, a feed spout 220, and the sanitization system 101.

In various embodiments, the sanitization system 101 comprises the ultraviolet light emitter 132 and/or the sanitization device 120 coupled to the feed spout 220. In various embodiments ultraviolet radiation emitted from the ultraviolet light emitter 132 and/or the sanitization device 120 is oriented / directed in a downward direction (i.e., towards a container 230 being filled, or in a direction of gravity). In this regard, the ultraviolet light emitter 132 and/or the sanitization device 120 can be configured to sanitize the container 230 being filled, a fluid being dispensed into the container 230, and/or a surface 240 after dispensing.

In various embodiments, the sanitization system 101 can be configured to sanitize as outlined previously herein during dispensing of a fluid, periodically, and/or after dispensing of a fluid. The present disclosure is not limited in this regard.

In various embodiments, the feed spout 220 comprises a handle 222 and a fluid outlet 224. In various embodiments, the handle 222 can mechanically open a valve to fluidly couple a fluid disposed in the housing 210 to the fluid outlet 224. In this regard, various mechanisms may be used as an input signal to the sanitization system 101 for sanitizing during dispensing. For example, pulling the handle 222 can close an electrical switch, coupling the power supply 150 to one of the ultraviolet light emitter 132 and/or the sanitization device 120, in accordance with various embodiments. In various embodiments, a flow sensor may be disposed in the feed spout 220 configured to detect mass flow rate of fluid therein. The controller 102 can use the flow sensor data to determine whether a fluid is being dispensed. In various embodiments, the handle 222 can be in mechanical communication with a piezoelectric sensor that sends the input signal 103 in response to the valve closing. In this regard, the sanitization process may commence after dispensing of the fluid disposed in the housing 210. Thus, one skilled in the art may recognize various initiation methods for sanitizing surrounding surfaces and/or containers (e.g., container 230 or server 140) during a beverage dispensing process and be within the scope of this disclosure.

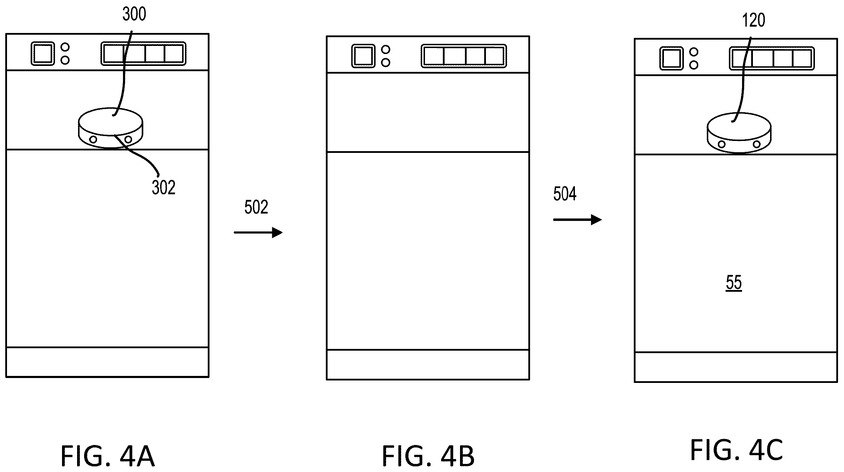

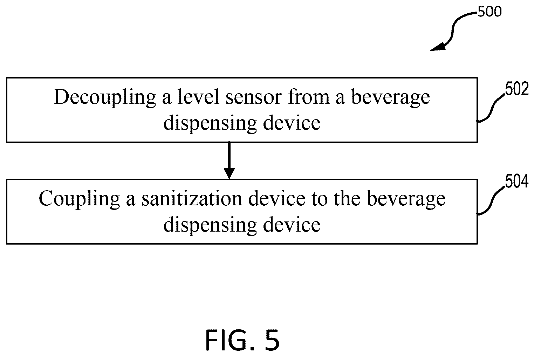

Referring now to FIGs. 4A, 4B, 4C, a retrofit process for integrating a sanitization system in a beverage maker is illustrated, in accordance with various embodiments. With combined reference to FIGs. 4A-C and 5, the retrofit process 500 comprises decoupling a level sensor 300 from a beverage device 55 (step 502). The level sensor 300 can comprise an infrared (IR) level sensor (e.g., an emitter configured to emit IR radiation between 780 nm and 1 mm) and an IR receiver. In various embodiments, the level sensor 300 comprises a housing 302. In various embodiments, the housing 302 defines a first shape (e.g., a partially annular shape, an annular shape, a cylindrical shape, or the like).

In various embodiments, the retrofit process 500 further comprises coupling a sanitization device 120 to the beverage dispensing device (step 504). In various embodiments, the sanitization device 120 is configured to detect a level of fluid in a container during dispensing of the fluid by the beverage dispensing device 55 and configured to sanitize the container during dispensing, the beverage dispensing device 55, or the like. In this regard, the sanitization device 120 comprises dual functionality as described previously herein. In various embodiments, the sanitization device comprises a second shape that is substantially similar to the first shape of the level sensor 300. "Substantially similar" as referred to herein includes remaining with a profile of 0.4 inches (1 cm) of a profile of the first shape, or within 0.2 inches (0.5 cm) or the like. In this regard, the sanitization device 120 can be installed in the same location as the level sensor 300 upgrading capability of the beverage maker with sanitization capabilities in an easy and efficient manner.

Benefits, other advantages, and solutions to problems have been described herein with regard to specific embodiments. Furthermore, the connecting lines shown in the various figures contained herein are intended to represent exemplary functional relationships and/or physical couplings between the various elements. The scope of the disclosure is accordingly to be limited by nothing other than the appended claims, in which reference to an element in the singular is not intended to mean "one and only one" unless explicitly so stated. Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

Systems, methods, and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

As used herein, the terms "comprises," "comprising," or any other variation thereof, are intended to cover a non-exclusive inclusion, such that a process, method, article, or apparatus that comprises a list of elements does not include only those elements but may include other elements not expressly listed.

Finally, it should be understood that any of the above described concepts can be used alone or in combination with any or all of the other above described concepts. Although various embodiments have been disclosed and described, one of ordinary skill in this art would recognize that certain modifications would come within the scope of the claims. Accordingly, the description is not intended to be exhaustive or to limit the principles described or illustrated herein to any precise form. Many modifications and variations are possible to the extent that the resulting embodiments fall within the scope of the claims.

## Claims

1. A beverage maker (55, 100), comprising:
a housing (110) defining a recess, the recess configured to receive a server in the recess; and
a sanitization device (120) disposed in the recess, the sanitization device comprising an ultraviolet light emitter (122) and an ultraviolet light sensor (124), the ultraviolet light emitter oriented towards a base of the housing, the sanitization device configured to detect a fluid level in the server during dispensing of a fluid and configured to at least partially sanitize one of the server and the fluid during dispensing of the fluid.

2. The beverage maker of claim 1, further comprising a controller (102) in electronic communication with the ultraviolet light emitter (122) and the ultraviolet light sensor (124), the controller configured to:
receive an input signal (103), wherein:
the input signal (103) is from one of a motion sensor or a weight sensor indicating that the server has been placed in the recess, or
the input signal (103) is from a button (104) disposed on the housing (110) being pressed;
couple a power supply (150) to the ultraviolet light emitter (122) in response to receiving the input signal;
receive sensor data from the ultraviolet light sensor (124); and
decouple the power supply from the ultraviolet light emitter in response to determining an ultraviolet radiation level exceeded an ultraviolet radiation threshold.

3. The beverage maker of claim 1, further comprising a second ultraviolet radiation emitter (132) disposed in the housing and oriented towards surfaces within the recess.

4. The beverage maker of claim 3, further comprising a controller (102) in electronic communication with the second ultraviolet radiation emitter (132), the controller configured to:
receive an input signal (103) from one of a button (106), a main controller of the beverage maker (100), or a motion sensor or a weight sensor;
couple a power supply (150) to the second ultraviolet radiation emitter in response to receiving the input signal; and
decouple the power supply (150) from the second ultraviolet radiation emitter in response to exceeding a time threshold.

5. The beverage maker of claim 3 or 4, wherein the ultraviolet light emitter and the second ultraviolet light emitter are each configured to emit ultraviolet radiation having an average wavelength between 200 nm and 280 nm.

6. A retrofit process (500) for a beverage dispensing device, the retrofit process comprising:
decoupling (502) a level sensor from a location of a housing of the beverage dispensing device, the level sensor configured to detect a level of a fluid in a container during a dispensing process of the beverage dispensing device; and
coupling (504) a sanitization device in the location of the housing, the sanitization device comprising an ultraviolet light emitter and an ultraviolet light sensor, the sanitization device configured to detect the level of the fluid in the container during the dispensing process of the beverage dispensing device and sanitize one of the beverage dispensing device and the container.

7. The retrofit process of claim 6, wherein the ultraviolet light emitter is configured to emit ultraviolet radiation having an average wavelength between 100 nm and 400 nm, and optionally
wherein the average wavelength is between 200 nm and 400 nm.

8. The retrofit process of claim 6 or 7, wherein the level sensor is configured to emit infrared radiation having an average wavelength between 780 nm and 1 mm.

9. The retrofit process of claim 8, wherein the level sensor comprises a first housing having a first shape and the sanitization device comprises a second housing having a second shape, and wherein, the second shape is substantially similar to the first shape, the second shape having a profile remaining within 1 cm (0.4 inches) of a profile of the first shape.

## Patentansprüche

1. Getränkezubereiter (55, 100), umfassend:
ein Gehäuse (110), das eine Aussparung definiert, wobei die Aussparung dazu konfiguriert ist, eine Serviereinrichtung in der Aussparung aufzunehmen; und
eine Desinfektionsvorrichtung (120), die in der Aussparung angeordnet ist, wobei die Desinfektionsvorrichtung einen Ultraviolettlichtemitter (122) und einen Ultraviolettlichtsensor (124) umfasst, wobei der Ultraviolettlichtemitter in Richtung einer Basis des Gehäuses ausgerichtet ist, wobei die Desinfektionsvorrichtung dazu konfiguriert ist, während eines Ausgebens eines Fluids einen Fluidfüllstand in der Serviereinrichtung zu erkennen, und dazu konfiguriert ist, während des Ausgebens des Fluids eines der Serviereinrichtung und des Fluids mindestens teilweise zu desinfizieren.

2. Getränkezubereiter nach Anspruch 1, ferner umfassend eine Steuerung (102) in elektronischer Verbindung mit dem Ultraviolettlichtemitter (122) und dem Ultraviolettlichtsensor (124), wobei die Steuerung zu Folgendem konfiguriert ist:
Empfangen eines Eingangssignals (103), wobei:
das Eingangssignal (103) von einem eines Bewegungssensors oder eines Gewichtssensors stammt und angibt, dass die Serviereinrichtung in die Aussparung gestellt wurde, oder
das Eingangssignal (103) von einer an dem Gehäuse (110) angeordneten Taste (104) stammt, die gedrückt wird;
Koppeln einer Stromversorgung (150) mit dem Ultraviolettlichtemitter (122) als Reaktion auf Empfangen des Eingangssignals;
Empfangen von Sensordaten von dem Ultraviolettlichtsensor (124); und
Entkoppeln der Stromversorgung von dem Ultraviolettlichtemitter als Reaktion auf Bestimmen, dass ein Ultraviolettstrahlungspegel einen Ultraviolettstrahlungsschwellenwert überschritten hat.

3. Getränkezubereiter nach Anspruch 1, ferner umfassend einen zweiten Ultraviolettstrahlungsemitter (132), der in dem Gehäuse angeordnet und auf Oberflächen innerhalb der Aussparung ausgerichtet ist.

4. Getränkezubereiter nach Anspruch 3, ferner umfassend eine Steuerung (102) in elektronischer Verbindung mit dem zweiten Ultraviolettstrahlungsemitter (132), wobei die Steuerung zu Folgendem konfiguriert ist:
Empfangen eines Eingangssignals (103) von einem einer Taste (106), einer Hauptsteuerung des Getränkezubereiters (100) oder eines Bewegungssensors oder eines Gewichtssensors;
Koppeln einer Stromversorgung (150) mit dem zweiten Ultraviolettstrahlungsemitter als Reaktion auf Empfangen des Eingangssignals; und
Entkoppeln der Stromversorgung (150) von dem zweiten Ultraviolettstrahlungsemitter als Reaktion auf Überschreiten eines Zeitschwellenwerts.

5. Getränkezubereiter nach Anspruch 3 oder 4, wobei der Ultraviolettlichtemitter und der zweite Ultraviolettlichtemitter jeweils dazu konfiguriert sind, Ultraviolettstrahlung mit einer durchschnittlichen Wellenlänge zwischen 200 nm und 280 nm zu emittieren.

6. Nachrüstprozess (500) für eine Getränkeausgabevorrichtung, wobei der Nachrüstprozess Folgendes umfasst:
Entkoppeln (502) eines Füllstandssensors von einer Stelle eines Gehäuses der Getränkeausgabevorrichtung, wobei der Füllstandssensor dazu konfiguriert ist, während eines Ausgabeprozesses der Getränkeausgabevorrichtung einen Füllstand eines Fluids in einem Behälter zu erfassen; und
Koppeln (504) einer Desinfektionsvorrichtung an der Stelle des Gehäuses, wobei die Desinfektionsvorrichtung einen Ultraviolettlichtemitter und einen Ultraviolettlichtsensor umfasst, wobei die Desinfektionsvorrichtung dazu konfiguriert ist, während des Ausgabeprozesses der Getränkeausgabevorrichtung den Füllstand des Fluids in dem Behälter zu erkennen und eines der Getränkeausgabevorrichtung und des Behälters zu desinfizieren.

7. Nachrüstprozess nach Anspruch 6, wobei der Ultraviolettlichtemitter dazu konfiguriert ist, Ultraviolettstrahlung mit einer durchschnittlichen Wellenlänge zwischen 100 nm und 400 nm zu emittieren, und optional wobei die durchschnittliche Wellenlänge zwischen 200 nm und 400 nm liegt.

8. Nachrüstprozess nach Anspruch 6 oder 7, wobei der Füllstandssensor dazu konfiguriert ist, Infrarotstrahlung mit einer durchschnittlichen Wellenlänge zwischen 780 nm und 1 mm zu emittieren.

9. Nachrüstprozess nach Anspruch 8, wobei der Füllstandssensor ein erstes Gehäuse mit einer ersten Form und die Desinfektionsvorrichtung ein zweites Gehäuse mit einer zweiten Form umfasst, und wobei die zweite Form im Wesentlichen der ersten Form ähnlich ist, wobei ein Profil der zweiten Form innerhalb von 1 cm (0,4 Zoll) von einem Profil der ersten Form bleibt.

## Revendications

1. Machine de préparation de boisson (55, 100), comprenant :
un boîtier (110) définissant un évidement, l'évidement étant configuré pour recevoir un serveur dans l'évidement ; et
un dispositif de désinfection (120) disposé dans l'évidement, le dispositif de désinfection comprenant un émetteur de lumière ultraviolette (122) et un capteur de lumière ultraviolette (124), l'émetteur de lumière ultraviolette étant orienté vers une base du boîtier, le dispositif de désinfection étant configuré pour détecter un niveau de fluide dans le serveur pendant la distribution d'un fluide, et configuré pour désinfecter au moins partiellement l'un du serveur et du fluide pendant la distribution du fluide.

2. Machine de préparation de boisson selon la revendication 1, comprenant également un contrôleur (102) en communication électronique avec l'émetteur de lumière ultraviolette (122) et le capteur de lumière ultraviolette (124), le contrôleur étant configuré pour :
recevoir un signal d'entrée (103), dans lequel :
le signal d'entrée (103) provient d'un capteur de mouvement ou d'un capteur de poids indiquant que le serveur a été placé dans l'évidement, ou
le signal d'entrée (103) provient d'un bouton (104) disposé sur le boîtier (110) en cours de pression ;
coupler une alimentation électrique (150) à l'émetteur de lumière ultraviolette (122) en réponse à la réception du signal d'entrée ;
recevoir des données de capteur provenant du capteur de lumière ultraviolette (124) ; et
découpler l'alimentation électrique de l'émetteur de lumière ultraviolette en réponse à la détermination d'un niveau de rayonnement ultraviolet dépassant un seuil de rayonnement ultraviolet.

3. Machine de préparation de boisson selon la revendication 1, comprenant également un second émetteur de rayonnement ultraviolet (132) disposé dans le boîtier et orienté vers les surfaces à l'intérieur de l'évidement.

4. Machine de préparation de boisson selon la revendication 3, comprenant également un contrôleur (102) en communication électronique avec le second émetteur de rayonnement ultraviolet (132), le contrôleur étant configuré pour :
recevoir un signal d'entrée (103) provenant d'un bouton (106), d'un contrôleur principal de la machine de préparation de boisson (100), ou d'un capteur de mouvement ou d'un capteur de poids ;
coupler une alimentation électrique (150) au second émetteur de rayonnement ultraviolet en réponse à la réception du signal d'entrée ; et
découpler l'alimentation électrique (150) du second émetteur de rayonnement ultraviolet en réponse au dépassement d'un seuil de temps.

5. Machine de préparation de boisson selon la revendication 3 ou 4, dans lequel l'émetteur de lumière ultraviolette et le second émetteur de lumière ultraviolette sont chacun configurés pour émettre un rayonnement ultraviolet ayant une longueur d'onde moyenne comprise entre 200 nm et 280 nm.

6. Procédé de modernisation (500) d'un dispositif de distribution de boisson, le procédé de modernisation comprenant :
le découplage (502) d'un capteur de niveau d'un emplacement d'un boîtier du dispositif de distribution de boisson, le capteur de niveau étant configuré pour détecter un niveau d'un fluide dans un récipient pendant un processus de distribution du dispositif de distribution de boisson ; et
le couplage (504) d'un dispositif de désinfection à l'emplacement du boîtier, le dispositif de désinfection comprenant un émetteur de lumière ultraviolette et un capteur de lumière ultraviolette, le dispositif de désinfection étant configuré pour détecter le niveau du fluide dans le récipient pendant le processus de distribution du dispositif de distribution de boisson et désinfecter l'un du dispositif de distribution de boisson et du récipient.

7. Procédé de modernisation selon la revendication 6, dans lequel l'émetteur de lumière ultraviolette est configuré pour émettre un rayonnement ultraviolet ayant une longueur d'onde moyenne comprise entre 100 nm et 400 nm, et éventuellement dans lequel la longueur d'onde moyenne est comprise entre 200 nm et 400 nm.

8. Procédé de modernisation selon la revendication 6 ou 7, dans lequel le capteur de niveau est configuré pour émettre un rayonnement infrarouge ayant une longueur d'onde moyenne comprise entre 780 nm et 1 mm.

9. Procédé de modernisation selon la revendication 8, dans lequel le capteur de niveau comprend un premier boîtier ayant une première forme et le dispositif de désinfection comprend un second boîtier ayant une seconde forme, et dans lequel la seconde forme est sensiblement similaire à la première forme, la seconde forme ayant un profil restant à moins de 1 cm (0.4 pouces) d'un profil de la première forme.
